# EUROPÄISCHE PATENTSCHRIFT

(11) **EP 2 632 507 B1**
(45) Veröffentlichungstag und Bekanntmachung des Hinweises auf die Patenterteilung: **24.09.2014**
(21) Anmeldenummer: 11771037.6
(22) Anmeldetag: 18.10.2011
(51) Int. Cl.: A61M 1/36, B01D 15/20, G01N 30/56

(54) **MEDIZINISCHE TRENNVORRICHTUNG**
MEDICAL SEPARATING DEVICE
DISPOSITIF DE SÉPARATION MÉDICAL

(30) Priorität: 29.10.2010 DE 102010049790
(43) Veröffentlichungstag der Anmeldung: 04.09.2013
(73) Patentinhaber: Fresenius Medical Care Deutschland GmbH, 61352 Bad Homburg (DE)
(72) Erfinder: LEINENBACH, Hans-Peter, A-3500 Krems-Rehberg (AT); GERNER, Franz-Josef, 66606 St. Wendel (DE); KUHN, Stefan, 66538 Neunkirchen (DE); SCHINZEL, Ekkehard, 66539 Neunkirchen (DE); PRIESNITZ, Patrick, 66606 St. Wendel (DE)
(74) Vertreter: Weiß, Stefan
(86) Internationale Anmeldenummer: PCT/EP2011/005218
(87) Internationale Veröffentlichungsnummer: WO 2012/055500

(56) Entgegenhaltungen:
- WO-A1-99/36110
- DE-A1-102008 053 131
- US-A1- 2005 145 573
- US-A1- 2007 276 351
- US-B1- 6 498 007

## Beschreibung

Die Erfindung betrifft medizinische Trennvorrichtungen, die aus einem hohlzylindrischen Gehäuse bestehen, wobei das Gehäuse an der Ober- und Unterseite verschlossen ist und über einen Fluidzulauf und einen Fluidablauf verfügt sowie über einen Einfüllstutzen für ein Trennmedium. Die Trennvorrichtung ist weiterhin geeignet zum Einsatz im technischen, analytischen oder pharmazeutischen Bereich.

Aus dem Stand der Technik sind verschiedene Behandlungsverfahren bekannt, bei denen medizinische Trennvorrichtungen, insbesondere Adsorber, zum Einsatz kommen. So werden in der Immun-Apherese vielfach Systeme eingesetzt, bei denen entweder kleinvolumige Gehäuse oder aufwändige größere Gehäuse aus Glas verwendet werden. Dabei können die eingesetzten Adsorber über eine radiale Zuleitung mit dem Trennmedium oder Adsorbermaterial befüllt und mit einem Verschlussstopfen verschlossen werden. Da die radiale Zuleitung aus dem Gehäuse herausragt, besteht die Gefahr, dass diese zum Beispiel durch Herunterfallen, Stosseinwirkung oder andere äußere zerstörerische Einflüsse beschädigt werden kann. Dies führt dazu, dass die Trennvorrichtung ihre Dichtigkeit und/oder Sterilität verlieren kann.

Nachteilig ist auch, dass der Verschlussstopfen nicht gegen eine unsachgemäße Handhabung geschützt ist, da die Möglichkeit besteht, den Verschlussstopfen aufgrund seiner leichten Zugänglichkeit zu öffnen. Dabei kann es zu einem Fehlanschluss des Adsorbers an die Patientenleitung kommen oder durch das Öffnen des Verschlussstopfens die Dichtigkeit und/oder Sterilität des Adsorbers verloren gehen.

Bei den bestehenden Systemen, die über einen radialen Befüllungsanschluss verfügen, wird dieser nach der Befüllung mit einem Verschlussstopfen verschlossen. Eine solche Konstruktion bedingt eine entsprechende Länge des Befüllungsanschlusses, damit die Befüllung mit dem Trennmedium und das Verschließen mit dem Stopfen gelingt. Daraus resultiert ein weiterer nachteiliger Aspekt, da das Verhältnis von Gehäuse zur Länge des Befüllungsanschlusses umso ungünstiger wird, je kleiner der Durchmesser des Gehäuses gewählt wird. Dies steht dem Bestreben nach einer kompakten Bauweise der Adsorbergehäuse entgegen. Eine kompakte Bauweise soll sich dadurch auszeichnen, dass die Trennvorrichtung einen möglichst geringen Platzbedarf, ein Mindestmaß an Umverpackung sowie an Transport- und Lagerraum benötigt.

Bei einigen Adsorbermodellen besteht außerdem die Gefahr, dass sich diese komplett auseinander bauen lassen, da die Schraubdeckel bzw. Bodenabdeckungen nicht gesondert gesichert sind. Dadurch kann die Originalität einer solchen Trennvorrichtung nicht über ihren gesamten Lebenszyklus sichergestellt werden.

Die dargelegten nachteiligen Faktoren führen dazu, dass die Sicherheit bei dem Einsatz der Trennvorrichtung am Patienten nicht zweifelsfrei garantiert werden kann.

Aus dem Stand der Technik sind weiterhin verschiedene Verfahren bekannt, wie Adsorber zusammen mit dem Trennmedium zur Anwendung kommen können.

So ist aus der DE 10 2008 053 131 A1 eine Anordnung zum Sterilisieren einer Endverpackung, insbesondere eines Adsorbers bekannt, bei der die Endverpackung über eine radiale Zuleitung mit einem Adsorbergehäuse verbunden ist. In der Endverpackung wird das Trennmedium bevorratet. Nach der Sterilisation bilden das Adsorbergehäuse und die Endverpackung über die radiale Zuleitung eine sterile Einheit. Diese Anordnung erlaubt, zu gegebener Zeit, die Überführung des Trennmediums in das Adsorbergehäuse in einer unsterilen Umgebung.

In einem weiteren Verfahren kann das Adsorbergehäuse direkt mit dem Trennmedium befüllt werden. Im Anschluss an die Befüllung ist eine Sterilisation der befüllten Trennvorrichtung mittels energiereicher, ionisierender Strahlung wie zum Beispiel alpha-, beta - oder gamma-Strahlung erforderlich.

Die EP 0 507 245 B1 beschreibt eine Vorrichtung zur Auftrennung von biologischen Materialien, bei der das Zulaufrohr für die aufzutrennende Lösung vorzugsweise seitlich angebracht ist.

Der Erfindung liegt nun die Aufgabe zugrunde, medizinische Trennvorrichtungen zur Verfügung zu stellen, wobei das Gehäuse der Trennvorrichtungen über einen seitlichen Einfüllstutzen für die Befüllung mit einem Trennmedium verfügt und einen kompakten Gehäuseaufbau erlaubt, was darüber hinaus zu einer Reduzierung an möglicher Umverpackung sowie Transport- und Lagerraum führt und somit zu einer Kosteneinsparung.

Die Erfindung soll sich darüber hinaus dadurch auszeichnen, dass durch konstruktive Elemente am Gehäuse dessen Originalität garantiert und somit die Sicherheit beim Einsatz der Trennvorrichtung für den Patienten erhöht wird.

Mit der erfindungsgemäßen Trennvorrichtung soll es weiterhin möglich sein, dass diese direkt mit dem Trennmedium befüllt und sterilisiert werden kann. Außerdem soll die Erfindung auch ein teilgefertigtes Gehäuse mit einem separaten Behältnis, welches das Trennmedium enthält, umfassen, so dass die finale Befüllung des Gehäuses nach der Sterilisation von Gehäuse, Schlauchsystem und Trennmedium in unsteriler Umgebung erfolgen kann.

Für die erfindungsgemäße Trennvorrichtung sollen die einzelnen Komponenten möglichst funktional konzipiert sein, um diese in einem schnellen und kostengünstigen Verfahren montieren zu können sowie dem Anwender eine einfach zu handhabende Trennvorrichtung zur Verfügung zu stellen.

Die Aufgabe wird erfindungsgemäß durch die Merkmale der Ansprüche 1, 8 und 11 dahingehend gelöst, dass der Einfüllstutzen für das Trennmaterial tangential am Gehäuse angebracht ist. Vorteilhafte Ausführungsformen der Erfindung sind Gegenstand der Unteransprüche.

Im Folgenden wird die erfindungsgemäße Vorrichtung unter Bezugnahme auf die Zeichnungen näher erläutert.
Figur 1 zeigt den äußeren Aufbau der Trennvorrichtung.
Figur 2 und Figur 2a zeigen den Innenaufbau der Trennvorrichtung mit seitlichem tangentialen Einfüllstutzen, Gehäuseendkappen und Halteelementen zur Befestigung der Gehäuseendkappen aus unterschiedlichen Perspektiven.
Figur 3 stellt einen Einrastmechanismus zum Verbinden der Gehäuseaußenwandsegmente dar.
Figur 4 zeigt eine Detaildarstellung der Halteelemente an der Gehäuseinnenwand und der Gehäuseendkappen mit umlaufender Rille oder Nut.
Figur 5 zeigt beispielhaft eine schematische Darstellung des Verfahrens zur Befüllung der Trennvorrichtung mit dem Trennmedium.

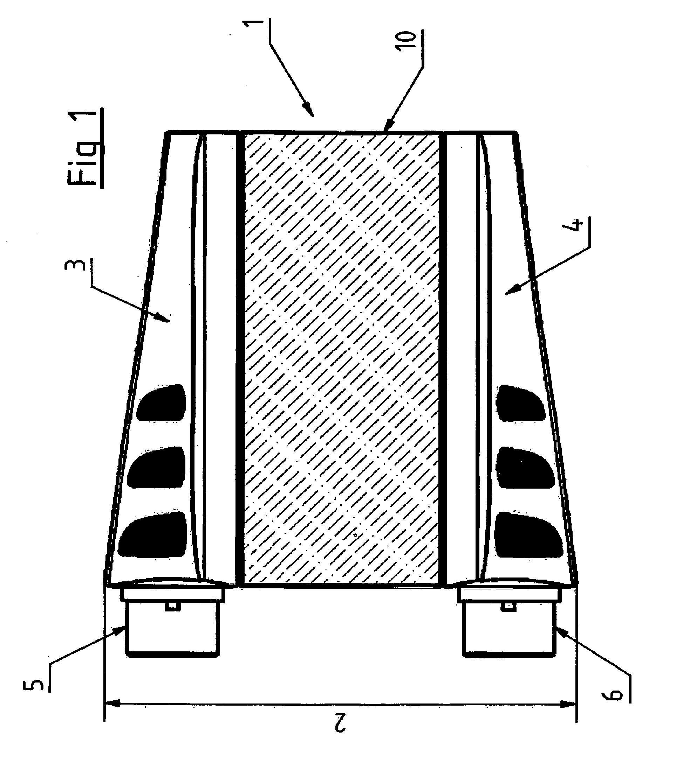

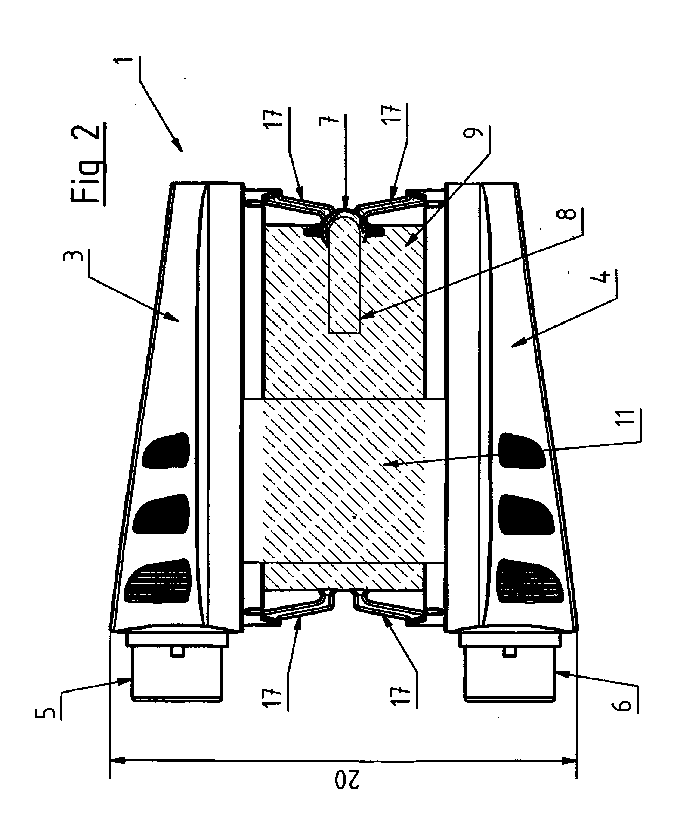

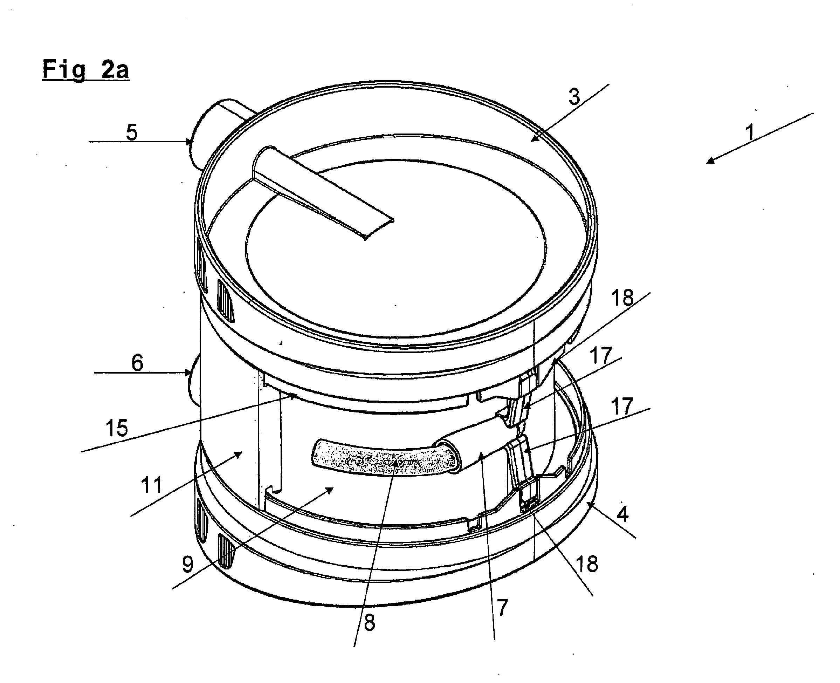

Der Aufbau der medizinischen Trennvorrichtung (1), wie in Figur 1, 2 und 2a dargestellt, bestehend aus einem hohlzylindrischen Gehäuse (2), welches an der Ober- und Unterseite durch Gehäuseendkappen (3, 4) verschlossen ist, über einen Fluidzulauf (5), einen Fluidablauf (6) und einen Einfüllstutzen (7) für die Befüllung der medizinischen Trennvorrichtung (1) mit dem Trennmedium, das auch als Adsorbens oder stationäre Phase bezeichnet werden kann, verfügt, wobei der Einfüllstutzen (7) tangential an der, im Wesentlichen runden, insbesondere zylindrisch, bauchig, konkav oder konvex ausgebildeten Gehäuse (9) der medizinischen Trennvorrichtung (1) angebracht ist.

Zur Herstellung einer Fluidverbindung zwischen einem mit dem Trennmedium gefüllten separaten Behältnis oder Vorratsbehälter (nicht dargestellt) und dem Einfüllstutzen (7) kann eine Schlauchleitung (8) in verschiedenster Weise an den Einfüllstutzen (7) angekoppelt werden. Vorzugsweise kann die Schlauchleitung (8) auf die Einfüllstutzen (7) geschweißt, aufgesteckt, geschraubt oder eingeschoben werden. Nach der Einfüllung des Trennmediums kann die Schlauchleitung (8) zwischen dem Behältnis und der erfindungsgemäßen Vorrichtung (1) in geeigneter Länge abgetrennt und verschlossen werden.

In einer bevorzugten Ausführungsform erlaubt eine seitliche, tangentiale Anordnung des Einfüllstutzens (7) insbesondere am Innengehäuse (9) eine kompakte und raumsparende Bauform.

Um eine solche Bauform der erfindungsgemäßen Trennvorrichtung (1) realisieren zu können, ragt der tangentiale Einfüllstutzen (7) um eine Länge zwischen 0 bis 20 mm, vorzugsweise 0 bis 18 mm, besonders bevorzugt 0 bis 16 mm aus dem Innengehäuse (9) heraus. Das Innengehäuse (9) selbst verfügt über einen Durchmesser von 3 bis15 cm, vorzugsweise von 4 bis 12 cm, besonders bevorzugt von 5 bis10 cm.

Damit das Innengehäuse (9) und der Einfüllstutzen (7) in einem ausgewogenen Verhältnis zueinander stehen, weist der Durchmesser des Innengehäuses (9) zur Länge des aus dem Innengehäuse (9) hervortretenden Einfüllstutzens (7) ein Verhältnis von bis zu 7,5/1, vorzugsweise bis zu 6,7/1, besonders bevorzugt bis zu 6,25/1 auf.

In einer weiteren bevorzugten Ausführungsform kann der an dem Einfüllstutzen (7) verbleibende Rest der Schlauchleitung (8) bequem um das Innengehäuse (9) gelegt werden.

Um den Einfüllstutzen (7) und die Schlauchleitung (8) gegenüber äußerer Einwirkung zu schützen, kann in einer besonders bevorzugten Ausführungsform einem Innengehäuse (9) eine Gehäuseaußenwand (10) vorgesetzt werden, was ebenfalls zur Kompaktheit und Sicherheit der medizinischen Trenneinrichtung (1) beiträgt.

Um das Innengehäuse (9) einschließlich des aus dem Innengehäuse (9) herausragenden Einfüllstutzens (7) durch eine Gehäuseaußenwand (10) umschließen zu können, beträgt der Abstand zwischen dem Innengehäuse (9) und der Gehäuseaußenwand (10) 2,0 bis 10 mm, vorzugsweise 3,5 bis 8 mm, besonders bevorzugt 4,5 bis 6,8 mm.

Die Gehäuseaußenwand (10) kann aus Gehäuseaußenwandsegmenten oder - paneelen (11) zusammengesetzt sein. Die Gehäuseaußenwand (10) besteht vorzugsweise aus 2, 4 oder 6 Gehäuseaußenwandsegmenten (11), die das Innengehäuse (9) der Trennvorrichtung (1) umschließen oder vorgesetzt sind.

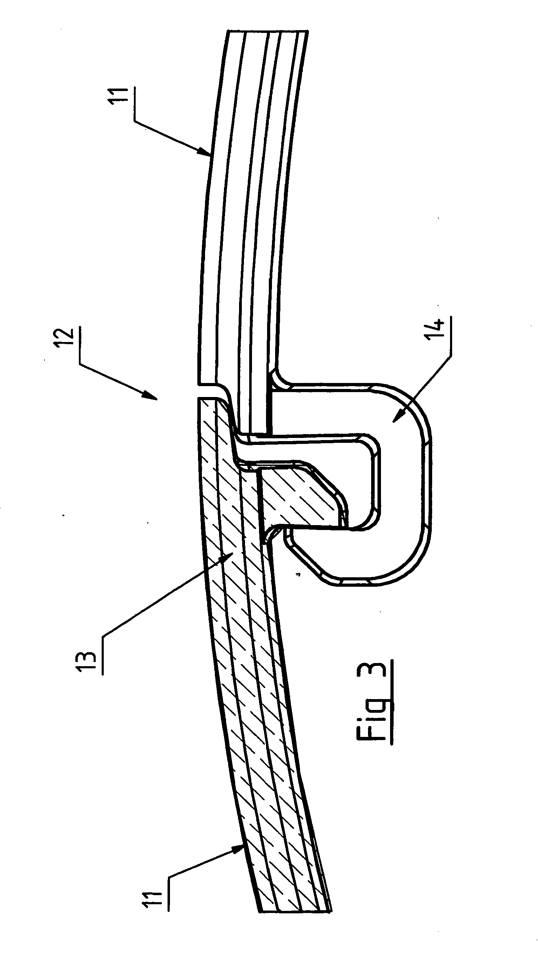

Um die einzelnen Gehäuseaußenwandsegmente (11) miteinander verbinden zu können, verfügen diese über einen Einrastmechanismus (12), wie in Figur 3 dargestellt, der sich vorzugsweise an den Außenseiten der Gehäuseaußenwandsegmente (11) befindet. Der Einrastmechanismus (12) kann in Form von Clip-, Schnapp-, oder Hakenverbindungen ausgebildet sein. Dabei versteht es sich von selbst, dass jedes Gehäuseaußenwandsegment (11) über mindestens ein Einrastelement (13) und mindestens ein Aufnahmeelement (14) verfügt.

Das Innengehäuse (9) einschließlich des Einfüllstutzens (7) und der Schlauchleitung (8), mittels einer Gehäuseaußenwand (10), vorzugsweise Gehäuseaußenwandsegmenten (11), zu verkleiden, bietet den Vorteil, dass an der Außenseite der erfindungsgemäßen Vorrichtung (1) keine radial abstehende Einfüllleitung oder Verschlusskappe mehr vorhanden ist. Sollte die Trennvorrichtung (1) einmal versehentlich fallengelassen werden, so liegt der Einfüllstützen (7) hinter der Gehäuseaußenwand (10) und ist vor Beschädigung, wie zum Beispiel dem Abbrechen oder Absplittern des Einfüllstutzens (7) oder eines Teils davon, geschützt.

Durch die Gehäuseaußenwand (10) ist die Trennvorrichtung (1) weiterhin vor anderen äußeren zerstörerischen Einflüssen wie zum Beispiel vor Stosseinwirkung geschützt. Dadurch, dass die Gehäuseaußenwand (10) ebenfalls die Schlauchleitung (8) verdeckt und somit über keine nach außen ragende Verschlusskappe mehr verfügt, wird eine unsachgemäße Handhabung wie zum Beispiel ein fehlerhaftes Anschließen der Trennvorrichtung (1) an der Patientenleitung oder das Entfernen der Verschlusskappe vermieden. Diese konstruktiven Maßnahmen gewährleisten die Originalität der Trennvorrichtung (1) und führen zu einer erhöhten Sicherheit beim Einsatz am Patienten.

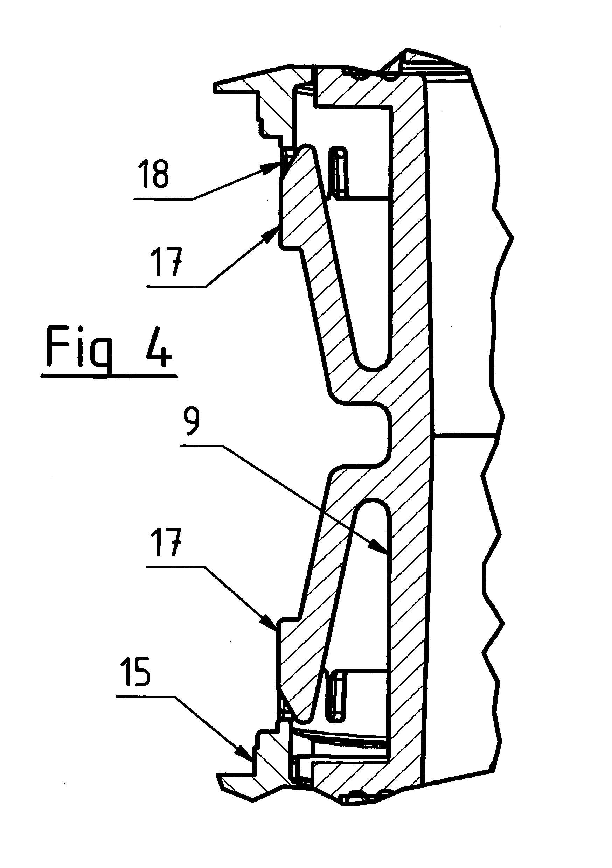

In einer weiteren besonders bevorzugten Ausführungsform kann die Gehäuseaußenwand (10), vorzugsweise die Gehäuseaußenwandsegmente (11), zwischen einer oberen und unteren Gehäuseendkappe (3, 4) eingeklemmt oder eingerastet werden. Dabei sind die Gehäuseendkappen (3, 4) zur Aufnahme der Gehäuseaußenwand (10), vorzugsweise der Gehäuseaußenwandsegmente (11), in Form einer umlaufenden Nut oder Rille (15) ausgebildet. Diese Ausführungsform kann den Figuren 2 und 4 entnommen werden.

In diesem Fall sind die Abmessungen des Innengehäuses (9) und des Einfüllstutzens (7) so zu wählen, dass sie zusammen kleiner sind als der Radius der Gehäuseendkappen (3, 4), so dass noch ausreichend Raum bleibt, um eine Gehäuseaußenwand (10) vorzusetzen. Die Größe der Gehäuseendkappen (3, 4) orientiert sich an den oben angegebenen Maßen der einzelnen Komponenten.

Bei der erfindungsgemäßen Trennvorrichtung (1) ist weiterhin vorgesehen, dass die Ober- und Unterseite der Trennvorrichtung (1) durch Gehäuseendkappen (3, 4) wie in den Figuren 1, 2 dargestellt verschlossen werden. Die Gehäuseendkappen (3, 4) können dabei auf des Gehäuse (2), vorzugsweise auf das Innengehäuse (9) aufgesteckt oder aufgeschraubt werden. Der Fluidzulauf (5) und der Fluidablauf (6) in den Gehäuseendkappen (3, 4) ist vorzugsweise radial angeordnet.

In einer bevorzugten Ausführungsform ist die Trennvorrichtung (1) derart ausgebildet, dass der radiale Fluidzulauf (5) und der radiale Fluidablauf (6) in eine vorbestimmte Richtung zeigen, besonders bevorzugt sind die Fluidanschlüsse (5, 6) übereinander angeordnet.

Für die Ausführungsform der Gehäuseendkappen (3, 4) mit Schraubverschluss ist die Position des Gewindeanfangs am Gehäuse (2) oder am Innengehäuse (9) und an den Gehäuseendkappen (3, 4) entscheidend. Die Position des Gewindeanfangs ist dabei so zu wählen, dass der radiale Fluidzulauf (5) und der radiale Fluidablauf (6) in eine vorbestimmte, vorzugsweise in eine übereinander angeordnete Richtung gebracht werden können.

Sofern die Gehäuseendkappen (3, 4) auf das Gehäuse (2) oder das Innengehäuse (9) aufgesteckt werden, ist die Aufsteckvorrichtung, gemäß den Kenntnissen des Fachmanns, dergestalt auszubilden, dass die oben beschriebene Ausrichtung der Fluidanschlüsse (5, 6) realisiert werden kann.

Eine zusätzliche verbesserte Sicherheit der Trennvorrichtung (1) lässt sich dadurch erzielen, dass bevorzugt am Innengehäuse (9) Halteelemente (17) zur Befestigung der oberen und unteren Gehäuseendkappen (3, 4) vorgesehen werden. Die Halteelemente (17) können in Form von Clip-, Schnapp-, oder Hakenverbindungen ausgebildet sein. Am Innengehäuse (9) sind wenigstens ein, vorzugsweise 2 oder mehr Halteelemente (17) vorgesehen, die besonders bevorzugt paarweise angebracht sind. Die Halteelemente (17) können darüber hinaus vertikal gegenüberliegend ausgerichtet sein. Gegenüber den Halteelementen (17) befinden sich in den Gehäuseendkappen (3, 4), vorzugsweise im Randbereich der Gehäuseendkappen (3, 4), Aufnahmeelemente (18) in Form von Aussparungen, Vertiefungen oder weiteren möglichen Ausgestaltungen, um die Gehäuseendkappen (3, 4) fest und sicher vorzugsweise am Innengehäuse (9) zu fixieren.

Die oben beschriebene Ausführungsform hat den Vorteil, dass die Originalität der Vorrichtung gewährleistet ist, da die Gehäuseendkappen (3, 4) nicht zerstörungsfrei von dem Gehäuse (2) oder dem Innengehäuse (9) entfernt werden können und damit auch die Sicherheit bei der Anwendung am Patienten erhöht wird.

Durch die Arretierung mittels der Halteelemente (17) am Gehäuse (2) vorzugsweise am Innengehäuse (9) und der Aufnahmeelemente (18) an den Gehäuseendkappen (3, 4) ist es ebenfalls möglich, den radialen Fluidzulauf (5) und den radialen Fluidablauf (6) in eine vorbestimmte Richtung auszurichten, vorzugsweise derart, dass der radiale Fluidzulauf (5) und der radiale Fluidablauf (6) übereinander angeordnet sind.

Eine solche Ausführungsform erlaubt eine einfache Montage und gleichzeitig eine verbesserte Dichtigkeit, da die einzelnen Teile der Vorrichtung (1) durch den Haltemechanismus eine gleichmäßige Vorspannung erfahren und sich die einzelnen Komponenten der Trennvorrichtung (1) mit einer hohen Dichtigkeit miteinander verbinden lassen.

Die oben beschriebenen Ausführungsformen führen zu einer Vereinfachung bei der Herstellung und der Montage, da baugleiche Teile verwendet werden können, die keine unterschiedlichen Anforderungen an die Montage der erfindungsgemäßen Trennvorrichtung (1) stellen und dem Anwender eine einfach zu handhabende Vorrichtung bietet, bei der der Anwender nicht auf eine besondere Einbaurichtung der Trennvorrichtung (1) im Gerät achten muss.

Im Weiteren umfasst die Erfindung ein Verfahren zur Herstellung eines Gehäuses für eine medizinische Trennvorrichtung (1), vorzugsweise eines Adsorbergehäuses mit einem tangential am Gehäuse angeordneten Einfüllstutzen (7), wobei der Einfüllstutzen (7) vorzugsweise seitlich am Innengehäuse (9) der Trennvorrichtung (1) angebracht ist. Das hohlzylindrische Gehäuse (2) wird mit einer oberen und unteren Gehäuseendkappe (3, 4) verschlossen, wobei die Gehäuseendkappen (3, 4) auf das Innengehäuse (9) aufgesteckt oder aufgeschraubt werden. Dabei ist das Schraubgewinde (16) derart ausgebildet, dass sowohl der radiale Fluidzulauf (5) als auch der radiale Fluidablauf (6), welche in den Gehäuseendkappen (3, 4) angeordnet sind, in eine vorbestimmte Richtung ausgerichtet werden können. Vorzugsweise werden die Gehäuseendkappen (3, 4) so montiert, dass der radiale Fluidzulauf (5) und der radiale Fluidablauf (6) übereinander stehen.

Um die Gehäuseendkappen (3, 4) fest und sicher montieren zu können, werden sie mit den Halteelementen (17) verbunden. Zur Fixierung der Gehäuseendkappen (3, 4) sind wenigstens ein, vorzugsweise 2 oder mehr Halteelemente (17) vorgesehen. Dabei ist es im Hinblick auf eine einfache Montage vorteilhaft, die Gehäuseendkappe (3, 4) derart aufzusetzen, dass sie sich mit den paarweise am Innengehäuse (9) vertikal gegenüberliegend angeordneten Halteelementen (17) verbinden lassen. Die Gehäuseendkappen (3, 4) werden durch die Halteelemente (17) in Form einer Clip-, Schnapp- oder Hakenverbindung am Innengehäuse (9) arretiert. Gegenüber den Halteelementen (17) befinden sich in den Gehäuseendkappen (3, 4), vorzugsweise im Randbereich der Gehäuseendkappen (3, 4), Aufnahmeelemente (18) in Form von Aussparungen, Vertiefungen oder anderen möglichen Ausgestaltungen, in welche die Haltelemente (17) eingepasst oder eingerastet werden können.

Dabei können die Haltelemente (17) und Aufnahmeelemente (18) in einer besonders bevorzugten Ausführungsform derart angebracht werden, dass die Ausrichtung des radialen Fluidzulaufs (5) und des radialen Fluidablaufs (6) in eine vorbestimmte Richtung zeigen, insbesondere derart, dass der radiale Fluidzulauf (5) und der radiale Fluidablauf (6) übereinander angeordnet sind.

In dem hohlzylindrischen Gehäuse (2) und/oder in den Gehäuseendkappen (3, 4) werden üblicherweise noch Trennelemente eingesetzt, bei denen es sich in der Regel um Siebe oder Netze handelt, die zur Stabilisierung auf einer Stützstruktur aufgebracht sein können und dazu dienen, das Trennmedium in einem vorgegebenen Raum zu begrenzen und eine möglichst optimale Fluidverteilung zu gewährleisten.

In einer ersten teilgefertigten Trennvorrichtung (20) bestehend aus Innengehäuse (19), Gehäuseendkappen (3, 4) und möglicherweise erforderlichen Trennelementen im Inneren der Trennvorrichtung (20), kann auf den Einfüllstutzen (7) eine Schlauchleitung montiert werden. Dabei kann die Schlauchleitung (8) im Sinne einer einfachen Verfahrensführung auf den Einfüllstutzen (7) geschweißt, aufgesteckt oder vorzugsweise eingeschoben werden. Selbstverständlich sind auch andere Varianten denkbar, um das Behältnis, in dem das Trennmedium bevorratet ist, mit dem Einfüllstutzen (7) des Innengehäuses (9) der teilgefertigten Trennvorrichtung (20) zu verbinden.

Nach der Befüllung der teilgefertigten Trennvorrichtung (20) mit dem Trennmedium wird die Schlauchleitung (8) in geeigneter Länge durchtrennt und verschlossen. Dabei kann das offene Ende der Schlauchleitung (8) mit einer Klemmeinrichtung, einem Stopfen oder in anderer geeigneter Weise mit Absperrorganen verschlossen werden. Im Hinblick auf eine kostengünstige und einfache Herstellung der teilgefertigten Trennvorrichtung (20) ist es vorzuziehen, die Schlauchleitung (8) durch ein Abschweißen zu durchtrennen und diese dadurch gleichzeitig steril zu verschließen. Der verbleibende Rest der Schlauchleitung (8), der sich noch an dem Einfüllstutzen (7) befindet, kann nun bequem um die Gehäuseinnenwand (9) gelegt werden. Vorzugsweise wird die Schlauchlänge so gewählt, dass sie vor dem nächstliegenden Haltelement (17) endet.

In einem weiteren Verfahrensschritt wird die Gehäuseaußenwand (10), vorzugsweise in Form von Gehäuseaußenwandsegmenten (11), um das Innengehäuse (9) angeordnet. Dabei ist in den Gehäuseendkappen (3, 4) vorzugsweise eine Rille oder Nut (15) vorgesehen, in die sich die Gehäuseaußenwand (10) oder die Gehäuseaußenwandsegmente (11) einpassen lassen. Vorzugsweise wird die Gehäuseaußenwand (10) aus mindestens 2 Gehäuseaußenwandsegmenten oder - paneelen (11) zusammengesetzt. Die einzelnen Gehäuseaußenwandsegmente (11) werden durch einen Einrastmechanismus (12) miteinander verbunden. Dazu sind, vorzugsweise an den Außenseiten der Gehäuseaußenwandsegmente (11), Einrastelemente (13) und Aufnahmeelemente (14) in Form von Clip-, Schnapp-, oder Hakenverbindungen (11) ausgebildet. Jedes einzelne Gehäuseaußenwandsegment (11) wird dabei wechselseitig sowohl über mindestens ein Einrastelement (13) als auch über mindestens ein Aufnahmeelement (14) miteinander verbunden und in die Gehäuseendkappen (3, 4) eingepasst oder vorzugsweise eingerastet. Zur Stabilisierung befinden sich an der Gehäuseaußenwand (10) oder den Gehäuseaußenwandsegmenten (11) in regelmäßigen Abständen Abstandselemente (19), die im Wesentlichen dem Abstand zwischen dem Innengehäuse (9) und der Gehäuseaußenwand (10) beziehungsweise den Außenwandsegmenten (11) entsprechen.

Die nun vollständig von der Gehäuseaußenwand (10) oder den Gehäuseaußenwandsegmenten (11) umschlossene Trennvorrichtung (1) wird abschließend einer Sterilisation mittels Dampf oder energiereicher, ionisierender Strahlung wie zum Beispiel UV, Röntgen-, alpha-, gamma- oder E-Beam-Strahlung unterzogen, sofern sie zum Beispiel als Adsorber zum Einsatz am Patienten vorgesehen ist.

In einer weiteren bevorzugten Ausführungsform ist die teilgefertigte Trennvorrichtung (20) so konzipiert, dass die Gehäuseaußenwand (10) aus 2 Gehäuseaußenwandsegmenten (11) besteht, wobei ein Gehäuseaußenwandsegment (11) bereits vormontiert ist, so dass nach der Befüllung mit dem Trennmedium und dem Abschweißen der Schlauchleitung (8) nur noch das letzte Gehäuseaußenwandsegment (11) eingepasst werden muss. Auch die auf diese Weise gefertigte Trennvorrichtung (1) ist, vor dem Einsatz am Patienten, einer Sterilisation wie oben beschrieben zu unterziehen.

In einer zweiten bevorzugten Ausführungsform kann das Trennmedium in einem separaten Kompartiment oder Behältnis (21) bevorratet und mit der teilgefertigten Trennvorrichtung (20) über eine Schlauchleitung (8) verbunden sein. Sowohl das mit dem Trennmedium gefüllte Behältnis (21) als auch die teilgefertigte Vorrichtung (26) können zunächst gemeinsam sterilisiert werden, wobei zu gegebener Zeit das Trennmedium in die teilgefertigte Vorrichtung (20) überführt werden kann. In diesem Fall kann das Trennmedium auch in einer unsterilen Umgebung in die teilgefertigte Trennvorrichtung (20) überführt werden, da die sterile Einheit aus Trennvorrichtung (20) und Behältnis (21) unangetastet bleiben. Nach dem Durchtrennen und Abschweißen der Schlauchleitung (8) kann auch hier wieder die Gehäuseaußenwand (10) oder das letzte Gehäuseaußenwandsegment (11) eingepasst werden.

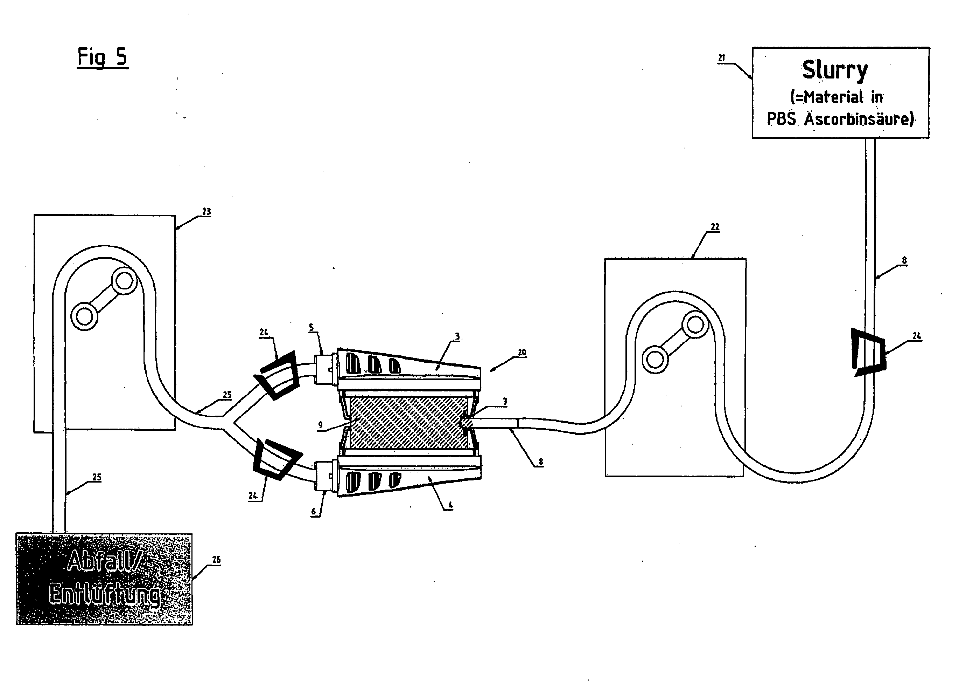

In Figur 5 ist beispielhaft das Füllverfahren einer teilgefertigten medizinischen Trennvorrichtung (20) dargestellt. Das Trennmedium kann in einem separaten Kompartiment oder Behältnis (21) vorliegen. Dieses ist über eine Schlauchleitung (8) mit dem tangential angeordneten Einfüllstutzen (7) am Innengehäuse (9) verbunden. Über eine Pumpe (22) wird das Adsorbermaterial oder Trennmedium in das Innengehäuse (9) der teilgefertigten Trennvorrichtung (20) gepumpt. Wenn der Füllvorgang beendet ist, wird der Fluidstrom (8) unterbrochen und die Pumpe (22) entfernt. Mit einer Pumpe (23) die über den Fluidzulauf (5) und/oder über den Fluidablauf (6) mit dem Innengehäuse (9) verbunden werden kann, wird die eventuell im Innengehäuse (9) befindliche Restluft entfernt, indem diese durch den Einfüllstutzen (7) herausgepumpt wird. Dadurch wird ein luftfreies Füllen des Gehäuses ermöglicht. Die Fluidwege können über die Schlauchklemmen (24) entsprechend geöffnet oder geschlossen werden. Anschließend wird die Schlauchleitung (8) an dem Einfüllstutzen (7) in geeigneter Länge durchtrennt und verschlossen. In einer bevorzugten Verfahrensführung wird die Schlauchleitung (8) durch Abschweißen durchtrennt und gleichzeitig steril verschlossen. Das Schlauchsystem (25) einschließlich der Pumpe (23) mit dem Behältnis (26) wird entfernt. Das an dem Einfüllstutzen (7) verbleibende Reststück der Schlauchleitung (8) wird um das Innengehäuse (19), vorzugsweise bis zum nächstliegenden Halteelement (17) gelegt und das letzte fehlende Gehäuseaußenwandsegment (11) eingesetzt.

Die erfindungsgemäße Trennvorrichtung (1) ist für die Verwendung im technischen, vorzugsweise analytischen, medizinischen und pharmazeutischen Bereich geeignet.

Die Trennvorrichtung (1) dient insbesondere zur Abreicherung von Substanzen aus einem Stoffgemisch, vorzugsweise zur Abreicherung von Peptiden oder Proteinen, besonders bevorzugt von Cytokinen, Low-Density-Lipoproteinen (LDL), toxischen Fremdproteinen wie zum Beispiel Tiertoxinen. Weiterhin dient die Vorrichtung (1) zur Abreicherung von Antikörpern, wobei es sich sowohl um körpereigene als auch um körperfremde, zum Beispiel therapeutisch wirksame Antikörper handeln kann. Die abzureichernden Substanzen können ebenfalls bakteriellen Ursprungs sein, sowohl von gramnegativen, als auch von grampositiven Bakterien wie zum Beispiel Endotoxine (Lipopolysaccharide) oder Enterotoxine zum Beispiel Toxic shock syndrom toxin -1 (TSST-1) sowie (Staphylococcus auraeus) sowie Staphylococcal enterotoxin B (SEB).

Besonders bevorzugt erfolgt die Abreicherung aus Vollblut oder Blutplasma.

Nachfolgend wird die erfindungsgemäße Vorrichtung (1) anhand eines nicht einschränkenden Beispiels mit den bevorzugten Abmessungen beschrieben.

Die Trennvorrichtung (1) bestand aus einem hohlzylindrischen Innengehäuse (9) mit einem Durchmesser im Mittel von 6,2 cm. Am Innengehäuse (9) war tangential ein Einfüllstützen (7) zur Befüllung der Trennvorrichtung (1) mit dem Trennmedium oder Adsorbens angeordnet. Der Einfüllstutzen (7) ragte mit einer Länge von 16,5 mm aus dem Innengehäuse (9) heraus. Zur Befüllung der Trennvorrichtung wurde eine Schlauchleitung (8) in den Einfüllstutzen (7) eingeschoben. An dem Innengehäuse (9) waren weiterhin paarweise zwei vertikal gegenüberliegende Halteelemente (17) in Form von Schnappverbindungen angebracht, die zur Arretierung der Gehäuseendkappen (3, 4) und zur Ausrichtung des radialen Fluidzulaufs (5) und des radialen Fluidablaufs (6) in eine übereinander angeordnete Richtung dienten. Dementsprechend waren in den Gehäuseendkappen (3, 4) Aufnahmeelemente (18) für die Halteelemente (17) angebracht. Die durchtrennte und verschweißte Schlauchleitung (8) reichte von ihrer Länge maximal bis zu den gegenüberliegenden Halteelementen (17). Das Innengehäuse (9) und die Schlauchleitung (8) wurden durch zwei Gehäuseaußenwandsegmente (11) umschlossen. Die beiden Gehäuseaußenwandsegmente (11) wurden über einen Einrastmechanismus (12) in Form einer Schnappverbindung miteinander verbunden, wobei jedes Gehäuseaußenwandsegment (11) über ein Einrastelement (13) und ein Aufnahmeelement (14) verfügte. Zwischen dem Innengehäuse (9) und den Gehäuseaußenwandsegmenten (11) lag ein Abstand zwischen 6.4 mm. Zur Stabilisierung der Gehäuseaußenwandsegmente (11) waren an deren Innenseite Abstandselemente angebracht, die im Wesentlichen dem Abstand zwischen dem Innengehäuse (9) und den Gehäuseaußenwandsegmenten (11) entsprachen.

## Patentansprüche

1. Medizinische Trennvorrichtung (1), umfassend
• ein an der Ober- und Unterseite verschlossenes rundes Gehäuse (9),
• einen Fluidzulauf (5),
• einen Fluidablauf (6) und
• einen Einfüllstutzen (7) für ein Trennmedium,
**dadurch gekennzeichnet, dass** der Einfüllstutzen (7) für das Trennmedium tangential an dem runden Gehäuse (9) der Trennvorrichtung (1) angebracht ist.

2. Medizinische Trennvorrichtung (1) nach Anspruch 1, **dadurch gekennzeichnet, dass** das runde Gehäuse (9) ein Innengehäuse ist.

3. Medizinische Trennvorrichtung (1) nach Anspruch 2, **dadurch gekennzeichnet, dass** der tangentiale Einfüllstutzen (7) seitlich am Innengehäuse (9) angebracht ist.

4. Medizinische Trennvorrichtung (1) nach Anspruch 1 oder 3, **dadurch gekennzeichnet, dass** an den tangentialen Einfüllstutzen (7) eine Schlauchstück (8) zur Befüllung der medizinischen Trennvorrichtung (1) angebracht ist.

5. Medizinische Trennvorrichtung (1) nach Anspruch 2, **dadurch gekennzeichnet, dass** die Ober- und Unterseite des runden Gehäuses (9) in Form von Gehäuseendkappen (3,4) ausgebildet sind, die auf das Innengehäuse (9) aufgesteckt oder aufgeschraubt werden und die Gehäuseendkappen (3,4) über einen radialen Fluidzulauf (5) und einen radialen Fluidablauf (6) verfügen.

6. Medizinische Trennvorrichtung (1) nach Anspruch 5, **dadurch gekennzeichnet, dass** die obere und untere Gehäuseendkappe (3, 4) derart auf dem Innengehäuse (9) angebracht sind, dass der radiale Fluidzulauf (5) und der radiale Fluidablauf (6) in eine vorbestimmte Richtung übereinander angeordnet sind.

7. Medizinische Trennvorrichtung (1) nach Anspruch 2, **dadurch gekennzeichnet, dass** eine Gehäuseaußenwand (10) oder Gehäuseaußenwandsegmente (11) aus mindestens 2, Gehäuseaußenwandsegmenten (11) bestehend, das Innengehäuse (9) umschließt oder vorgesetzt wird.

8. Verfahren zur Herstellung einer medizinischen Trenneinrichtung, **dadurch gekennzeichnet, dass**
• die medizinische Trennvorrichtung über ein Schlauchstück (8) am tangential angeordneten Einfüllstutzen (7) mit einem Trennmedium befüllt,
• das Schlauchstück (8) an dem tangentialen Einfüllstutzen (7) nach der Befüllung mit dem Trennmedium durchtrennt und mittels Absperrorganen verschlossen und
• der verbleibende Rest des Schlauchstücks (8) um das Innengehäuse (9), welches als rundes Gehäuse ausgebildet ist, gelegt wird.

9. Verfahren nach Anspruch 8, **dadurch gekennzeichnet, dass** dem Innengehäuse (9) anschließend eine Gehäuseaußenwand (10) oder Gehäuseaußenwandsegmente (11), vorgesetzt wird.

10. Verfahren nach Anspruch 9, **dadurch gekennzeichnet, dass** ein oder mehrere Gehäuseaußenwandsegmente (11) in einer teilgefertigten medizinischen Trennvorrichtung (20) vormontiert sind.

11. Verwendung der Vorrichtung (1) gemäß Anspruch 1 im technischen, analytischen, medizinischen und pharmazeutischen Bereich.

12. Verwendung der Vorrichtung (1) gemäß Anspruch 11 zur Abreicherung von Substanzen aus einem Stoffgemisch.

13. Verwendung der Vorrichtung (1) gemäß Anspruch 12, **dadurch gekennzeichnet, dass** es sich bei den Substanzen zur Abreicherung aus einem Stoffgemisch um Peptide oder Proteine handelt.

14. Verwendung der Vorrichtung (1) gemäß Anspruch 12, **dadurch gekennzeichnet, dass** es sich bei den Substanzen zur Abreicherung aus einem Stoffgemisch um Cytokine, Low-Density-Lipoproteine (LDL), toxische Fremdproteine, Antikörper, Endotoxinen und/oder Enterotoxinen handelt.

15. Verwendung der Vorrichtung (1) gemäß Anspruch 12, **dadurch gekennzeichnet, dass** die Abreicherung aus Vollblut oder Blutplasma erfolgt.

## Claims

1. A medical separation device (1), comprising
• a round housing (9), which is closed at the top side and at the bottom side,
• a fluid inlet (5),
• a fluid outlet (6), and
• a filling connection (7) for a separation medium,
**characterized in that** the filling connection (7) for the separation medium is attached tangentially to the round housing (9) of the separation device (1) .

2. The medical separation device (1) according to claim 1, **characterized in that** the round housing (9) is an interior housing.

3. The medical separation device (1) according to claim 2, **characterized in that** the tangential filling connection (7) is mounted on the side of the interior housing (9).

4. The medical separation device (1) according to claim 1 or 3, **characterized in that** a length of tubing (8) for filling the medical separation device (1) is mounted on the tangential filling connection (7) .

5. The medical separation device (1) according to claim 2, **characterized in that** the top and bottom sides of the round housing (9) are designed in the form of housing end caps (3, 4), which are placed on or screwed onto the interior housing (9), and the housing end caps (3, 4) have a radial fluid inlet (5) and a radial fluid outlet (6).

6. The medical separation device (1) according to claim 5, **characterized in that** the top and bottom housing end caps (3, 4) are attached to the interior housing (9) in such a way that the radial fluid inlet (5) and the radial fluid outlet (6) are situated one above the other in a predetermined direction.

7. The medical separation device (1) according to claim 2, **characterized in that** an exterior housing wall (10) or housing exterior wall segments (11) consisting of at least two exterior housing wall segments (11) encloses or is placed in front of the interior housing (9).

8. A method for producing a medical separation device, **characterized in that**
• the medical separation device is filled with a separation medium through a length of tubing (8) on the filling connection (7) situated tangentially,
• the length of tubing (8) on the tangential filling connection (7) is separated after filling with the separation medium and is sealed by means of cutoff elements, and
• the remainder of the length of tubing (8) is placed around the interior housing (9), which is designed as a round housing.

9. The method according to claim 8, **characterized in that** an exterior housing wall (10) or housing exterior wall segments (11) is placed in front of and adjacent to the interior housing (9).

10. The method according to claim 9, **characterized in that** one or more housing exterior wall segments (11) are premounted in a partially fabricated medical separation device (20).

11. Use of the device (1) according to claim 1 in the technical, analytical, medical and pharmaceutical fields.

12. Use of the device (1) according to claim 11 for reducing the concentration of substances in a substance mixture.

13. Use of the device (1) according to claim 12, **characterized in that** the substances whose concentration is to be reduced from a substance mixture are peptides or proteins.

14. Use of the device (1) according to claim 12, **characterized in that** the substances whose concentration is to be reduced from a substance mixture are cytokines, low-density lipoproteins (LDL), toxic foreign proteins, antibodies, endotoxins and/or enterotoxins.

15. Use of the device (1) according to claim 12, **characterized in that** the concentration in whole blood or blood plasma is reduced.

## Revendications

1. Dispositif de séparation médical (1), comprenant
• un boîtier rond (9) fermé sur le dessus et le dessous,
• une arrivée de fluide (5),
• une évacuation de fluide (6) et
• une tubulure de remplissage (7) pour un agent de séparation,
**caractérisé en ce que** la tubulure de remplissage (7) pour l'agent de séparation est appliquée en étant tangentielle sur le boîtier rond (9) du dispositif de séparation (1).

2. Dispositif de séparation médical (1) selon la revendication 1, **caractérisé en ce que** le boîtier rond (9) est un boîtier intérieur.

3. Dispositif de séparation médical (1) selon la revendication 2, **caractérisé en ce que** la tubulure de remplissage (7) tangentielle est placée sur le côté du boîtier intérieur (9).

4. Dispositif de séparation médical (1) selon la revendication 1 ou 3, **caractérisé en ce qu'**une pièce de tuyau (8) pour remplir le dispositif de séparation médical (1) est placée sur la tubulure de remplissage (7) tangentielle.

5. Dispositif de séparation médical (1) selon la revendication 2, **caractérisé en ce que** le dessus et le dessous du boîtier rond (9) ont la forme de bouchons d'extrémité de boîtier (3, 4) qui sont placés ou vissés sur le boîtier intérieur (9) et les bouchons d'extrémité de boîtier (3, 4) disposent d'une arrivée de fluide (5) radiale et d'une évacuation de fluide (6) radiale.

6. Dispositif de séparation médical (1) selon la revendication 5, **caractérisé en ce que** les bouchons d'extrémité de boîtier (3, 4) supérieur et inférieur sont ainsi appliqués sur le boîtier intérieur (9) que l'arrivée de fluide (5) radiale et l'évacuation de fluide (6) radiale sont disposées l'une au-dessus de l' autre dans un sens prédéterminé.

7. Dispositif de séparation médical (1) selon la revendication 2, **caractérisé en ce qu'**une paroi extérieure de boîtier (10) ou des segments de paroi extérieure de boîtier (11) se composant d'au moins 2 segments de paroi extérieure de boîtier (11), ferment, ou sont placés devant, le boîtier intérieur (9).

8. Procédé de fabrication d'un dispositif de séparation médical, **caractérisé en ce que**
• le dispositif de séparation médical est rempli d'un agent de séparation sur la tubulure de remplissage (7) tangentielle via une pièce de tuyau (8),
• la pièce de tuyau (8) sur la tubulure de remplissage (7) tangentielle se sépare de l'agent de séparation après le remplissage et est fermée par des organes d'arrêt et
• le morceau restant de la pièce de tuyau (8) est placé autour du boîtier intérieur (9) qui est formé en tant que boîtier rond.

9. Procédé selon la revendication 8, **caractérisé en ce qu'**une paroi extérieure de boîtier (10) ou des segments de paroi extérieure de boîtier (11) sont ensuite placés devant le boîtier intérieur (9).

10. Procédé selon la revendication 9, **caractérisé en ce qu'**un ou plusieurs segments de paroi extérieure de boîtier (11) sont prémontés dans un dispositif de séparation médical (20) partiellement fabriqué.

11. Emploi du dispositif (1) selon la revendication 1 dans le domaine technique, analytique, médical et pharmaceutique.

12. Emploi du dispositif (1) selon la revendication 11, pour appauvrir des substances d'un mélange de substances.

13. Emploi du dispositif (1) selon la revendication 12, **caractérisé en ce que** les substances pour l'appauvrissement d'un mélange de substances, sont des peptides et des protéines.

14. Emploi du dispositif (1) selon la revendication 12, **caractérisé en ce que** les substances pour l'appauvrissement d'un mélange de substances, sont des cytokines, des lipoprotéines de faible densité (LDL), des protéines étrangères toxiques, des anticorps, des endotoxines et/ou des entérotoxines.

15. Emploi du dispositif (1) selon la revendication 12, **caractérisé en ce que** l'appauvrissement s'effectue à partir de sang total ou de plasma sanguin.
